# NOUVEAU FASCICULE DE BREVET EUROPEEN

(11) **EP 0 835 647 B2**
(45) Date de publication et mention de la décision concernant l'opposition: **17.03.2010**
(45) Mention de la délivrance du brevet: 06.10.1999
(21) Numéro de dépôt: 97402251.9
(22) Date de dépôt: 26.09.1997
(51) Int. Cl.: A61K 8/04, A61K 8/73, A61K 8/81, A61K 8/92, A61Q 19/00

(54) **Crème auto-moussante**
Selbstschäumende Creme
Self-foaming cream

(30) Priorité: 14.10.1996 FR 9612510
(43) Date de publication de la demande: 15.04.1998
(73) Titulaire: L'Oréal, 75008 Paris (FR)
(72) Inventeur: Touzan, Philippe, 75012 Paris (FR); Delambre, Patricia, 94480 Ablon/S/Seine (FR)
(74) Mandataire: Catherine, Alain

(56) Documents cités:
- EP-A- 0 663 203
- FR-A- 2 698 004
- BROCHURE "PEMULEN POLYMERIC EMULSIFIERS" (2354-90-41B) 14 Mai 1990,
- CTFA INTERNATIONAL COSMETIC INGREDIENT DICTIONARY, vol. 4ÈME ED, 1991, page 12
- BROCHURE "PEMULEN POLYMERIC EMULSIFIERS" (2354-90-38B) 14 Mai 1990,
- BROCHURE "SEPIGEL*305", Mai 1993,

## Description

L'invention a pour objet des dispositifs aerosols pressurisés à post moussage comprenant des crèmes auto-moussantes se présentant sous la forme d'une émulsion huile-dans-eau pressurisable. Ces crèmes à usage topique ont un bon pouvoir moussant et sont destinées plus particulièrement au nettoyage et au soin de la peau.

Le nettoyage de la peau est très important pour le soin du visage. Il doit être le plus performant possible car les résidus gras tels que l'excès de sébum, les restes des produits cosmétiques utilisés quotidiennement et les produits de maquillage, notamment les produits «waterproof» résistants à l'eau, s'accumulent dans les replis cutanés et à la surface de la peau et peuvent obstruer les pores de la peau, entraîner l'apparition de boutons. Une mauvaise qualité de nettoyage est souvent responsable, parmi d'autres facteurs de cause, d'un teint brouillé.

On connaît plusieurs grands types de produits de nettoyage de la peau : les lotions et les gels aqueux détergents moussants, les huiles et les gels anhydres nettoyants rinçables, les laits démaquillants, les crèmes moussantes qui sont habituellement à base de savon.

Les lotions et les gels aqueux détergents moussants ont une action nettoyante grâce aux tensioactifs qui mettent en suspension les résidus gras et les pigments des produits de maquillage. Ils sont efficaces et agréables cosmétiquement du fait qu'ils moussent et qu'ils sont facilement éliminés. Dans la mesure où ils ne contiennent pas d'huile cosmétique, ils présentent l'inconvénient d'assécher la peau par leur action délipidante. C'est le cas par exemple des produits décrits par le document WO-A-95/05769, qui enseigne des lotions pour le nettoyage de la peau, très fluides, pressurisables, produisant une belle mousse, mais ces produits détruisent le film hydro-lipidique de la peau et laissent la peau propre mais rêche.

Les huiles et les gels anhydres rinçables ont une action nettoyante grâce aux huiles contenues dans ces formulations. Ces huiles permettent la solubilisation des résidus gras et la dispersion des pigments de maquillage. Ces produits sont efficaces et bien tolérés. Ils présentent l'inconvénient d'être lourds, de ne pas mousser et de ne pas conférer de sensation de fraîcheur à l'application, ce qui est pénalisant d'un point de vue cosmétique.

Les crèmes et les laits démaquillants contiennent à la fois des huiles, des émulsionnants et des tensioactifs détergents en quantité suffisamment faible pour ne pas déstabiliser l'émulsion. Ces produits en dépit de leur bonne efficacité ne sont pas moussants et présentent une rinçabilité insuffisante qui nécessite l'emploi d'une lotion tonique détergente complémentaire pour parfaire le rinçage et l'élimination des salissures. Outre son caractère astreignant l'utilisation de ce deuxième produit peut entraîner à long terme un assèchement de la peau.

On a cherché à concevoir des produits moussants nettoyants, parfaitement rinçables à l'eau, contenant peu
ou pas de tensioactifs et susceptibles de comprendre des huiles en quantités élevées si on le souhaite, de façon à rendre optimal le nettoyage de la peau et à hydrater et nourrir celle-ci en évitant tout phénomène d'assèchement et d'irritation. On a cherché à obtenir une crème ayant l'efficacité d'une crème au savon (moussage et pouvoir nettoyant) ne comportant pas les inconvénients de ces dernières, en particulier le décapage de la peau qui est lié à un fort pourcentage de tensioactifs moussants.

Pour réaliser un tel produit, on ne peut pas simplement introduire des huiles dans une lotion ou un gel aqueux. En effet, les huiles ont tendance à inhiber les propriétés moussantes de ces formulations ; on dit que les huiles «tuent» la mousse. De plus, la dispersion de l'huile est instable. On connaît, par exemple par le document WO-A-95/17163, des émulsions moussantes, par exemple des émulsions démaquillantes. Ce type de produit est très doux, très bien toléré par la peau, mais le pouvoir moussant de ces émulsions est faible à cause de la présence des huiles. En outre, ces émulsions sont relativement épaisses, ce qui ne permet pas de les conditionner dans des récipients pressurisés.

Par ailleurs, on sait, par exemple par le document WO-A-89111907, que la méthode d'inversion de phase permet de préparer des émulsions huile-dans-eau, fluides, stables. Mais les émulsions préparées par la méthode d'inversion de phase dans l'art antérieur sont non-moussantes et sont restituées sous forme de spray lorsqu'elles sont pressurisées. En outre, elles comprennent des quantités de tensioactifs non négligeables.

On connaît par le document US-A-4,808,388 des crèmes sous la forme d'émulsions huile-dans-eau, pressurisées, moussantes, comprenant au moins 2 % d'un tensioactif non-ionique, jusqu'à 21 % d'huile et de 0,5 à 4,5 % d'un agent de consistance. De telles compositions ne sont restituées de façon satisfaisante (brillance, fermeté, densité) qu'à la condition d'employer CO₂ ou N₂O comme gaz propulseur.

On a cherché à préparer des compositions dont la tolérance est améliorée par rapport à celles de l'art antérieur, se présentant sous la forme de crèmes auto-moussantes pressurisables comprenant moins de 2 % de tensioactifs, et éventuellement pas de tensioactifs du tout.

Aussi, c'est avec étonnement que la demanderesse a remédié aux inconvénients de l'art antérieur grâce à des dispositifs aerosols comprenant des crèmes auto-moussantes, sous la forme d'une émulsion huile-dans-eau, pressurisable, qui, pressurisée, donne un post-moussage. Ces crèmes ont un bon pouvoir moussant, tout en comprenant peu ou pas de tensioactifs.

L'invention a pour objet un dispositif aérosol constitué d'un récipient pressurisable muni d'un moyen de diffusion comprenant une valve, ledit récipient comprenant un moyen de propulsion et une composition auto-moussante, sous la forme d'une émulsion huile-dans-eau, pressurisée et conditionnée dans le récipient aérosol, à post-moussage, comprenant :
(A) 10 à 30% en poids par rapport au poids total de la composition, d'une phase grasse comprenant au moins une huile cosmétique ;
(B) de 0,1 à 2% d'un système gélifiant comprenant :
   - (B1) au moins un polymère émulsionnant choisi parmi:
      (1) les homopolymères réticulés, formés à partir d'au moins un monomère, cationique ou anionique, à insaturation éthylènique choisi parmi le (meth)acrylate d'ammonium; l'acide 2-acrylamido-2-méthylpropane sulfonique ainsi que ses sels ; les dialkylaminoalkyl-(meth)acrylates, de préférence le diméthylamino-éthylmethacrylate ainsi que leurs sels quaternaires ou acides ; les dialkyl-aminoalkyl-(meth)acrylamides ainsi que leurs sels quaternaires ou acides ; les radicaux alkyle étant, de préférence, en C₁-C₄, et d'un agent de réticulation a polyinsaturation éthylénique,
      (2) les copolymères réticulés formés à partir d'au moins un monomère, cationique ou anionique, à insaturation éthylènique, d'au moins un co-monomère non-ionique et d'un agent de réticulation à polyinsaturation éthylènique, et
   - (B2) au moins un gélifiant hydrophile naturel ou synthétique ;
(C) de l'eau.

Par 'auto-moussante', on entend une composition susceptible de donner lieu à la formation d'une mousse et qui contient moins de 2 % de tensioactifs. Par 'post-moussage', on entend une composition qui, lorsqu'elle est pressurisée, est restituée sous la forme d'une crème, cette crème moussant spontanément lorsqu'on l'étale ou la manipule sur la peau.

Lorsque l'on exerce une pression sur le moyen de diffusion, on actionne la valve et le dispositif restitue son contenu sous la forme d'une crème onctueuse. A l'application sur la peau, cette crème se transforme en une mousse dense, fine, onctueuse, abondante et ferme. Des produits d'une telle consistance sont particulièrement appréciés pour le nettoyage et le soin de la peau ou des cheveux.

Les compositions à post-moussage sont tout à fait distinctes des compositions moussantes pressurisées classiques qui sont distribuées sous la forme d'une mousse, comme c'est le cas par exemple des compositions décrites dans US-A-4,808,388.

Les copolymères réticulés utilisés dans les compositions selon l'invention comprennent au moins un monomère cationique ou anionique à insaturation éthylénique, au moins un co-monomère non-ionique et un agent de réticulation à polyinsaturation éthylénique.
Le monomère anionique est choisi en particulier parmi l'acide (meth)acrylique, le (meth)acrylate d'ammonium et l'acide 2-acrylamido-2-méthylpropane sulfonique ainsi que ses sels.
Le monomère cationique est choisi en particulier parmi les dialkylaminoalkyl(meth)acrylates, de préférence le diméthylamino-éthylmethacrylate ; les dialkylaminoalkyl-(meth)acrylamides ainsi que leurs sels quaternaires ou acides : les radicaux alkyle étant, de préférence, en C₁-C₄.
Le co-monomère non-ionique est choisi en particulier parmi le méthacrylamide, l'acrylamide, les esters d'acide (meth)acrylique en C₁₀-C₃₀, les esters de vinyle.

Les agents de réticulation à polyinsaturation éthylénique sont choisis, de préférence, parmi le divinylbenzène; le tétraallyloxyéthane; l'éther diallylique; les éthers polyallyliques polyglycéryliques; les éthers allyliques d'alcools de la série du sucre comme l'érythrytol, le pentaérythrytol, l'arabitol, le sorbitol ou le glucose; le méthylène-bis-acrylamide, l'éthylèneglycol di-(méthyl)acrylate, le di-(meth)acrylamide, le cyano-méthylacrylate, le vinyloxyéthyl-(meth)acrylate ou leurs sels métalliques.

Les polymères émulsionnants du système gélifiant (B) utilisés dans les compositions selon l'invention sont choisis préférentiellement parmi:
(a) les copolymères comportant une fraction majoritaire d'acide acrylique et une faible fraction d'esters d'acide (meth)acrylique en C₁₀-C₃₀, tels que les produits vendus sous les noms PEMULEN TR1, PEMULEN TR2 et CAR-BOPOL 1342 par la société GOOORICH (ils sont décrits et préparés dans le document EP-A-268 164) ;
(b) les copolymères d'acide 2-acrylamido-2-méthyl-propane sulfonique partiellement ou totalement neutralisé (par une base telle que la soude, de la potasse ou une amine) et d'acrylamide, tels que la produit décrit dans l'exemple 1 du document EP-A-503 853 ;
(c) les homopolymères de diméthylaminoéthyl-méthacrylate quaternisé par le chlorure de méthyle, tels que les produits vendus sous les noms SALCARE 95 et SALCARE 96 par la société ALLIED COLLOIDS ou les copolymères de diméthylamino-éthylméthacrylate quaternisé par le chlorure de méthyle et d'acrylamide tel que le produit SALCARE SC92 vendu par ALLIED COLLOIDS ou le produit PAS 5194 vendu par HOECHST (ils sont décrits et préparés dans le document EP-A-395.282),
(d) les copolymères d'acide (méth)acrylique et d'esters de vinyle tels que le copolymère d'acide acrylique et d'isodécanoate de vinyle, par exemple les produits vendus sous les dénominations STABYLEN 30 et PNC 300 par la société 3V.

Les homopolymères ou copolymères réticulés utilisés dans les compositions de l'invention sont plus particulièrement choisis parmi ceux définis dans les paragraphes (a) et (b) précédents.

Le gélifiant hydrophile naturel ou synthétique du système gélifiant (B) peut être choisi parmi les gommes naturelles ou modifiées et parmi les polymères synthétiques hydrophiles ayant des propriétés gélifiantes.

Par gomme naturelle ou modifiée, on entend un polysaccharide éventuellement modifié qui s'hydrate en milieu aqueux pour former une solution visqueuse ou une dispersion. Parmi les gommes naturelles, on inclut les extraits d'algues, les exsudats de plantes et les gommes extraites de graines ou de racines végétales et celles obtenues par fermentation microbiologique. Les gommes modifiées ou semi-synthétiques comprennent les dérivés de la cellulose et de l'amidon et, de façon générale, les dérivés de toutes les gommes naturelles. Sont inclus dans la définition des gommes selon la présente invention tous les composés cités dans les documents suivants :
- « GUMS, Encyclopedia of Chemical Technology, KIRK-OTHMER», vol.12, p.842-862, 4ème édition, 1994, Wiley.
- « STARCH, Encyclopedia of Chemical Technology, KIRK-OTHMER», vol.21, p.492-507, 3ème édition, 1985, Wiley.
- « CELLULOSE ESTERS, Encyclopedia of Chemical Technology, KIRK-OTHMER», vol.5, p.496-540, 4ème édition, 1993, Wiley.
- « CELLULOSE ETHERS, Encyclopedia of Chemical Technology, KIRK-OTHMER», vol.5, p.541-563, 4ème édition, 1993, Wiley.

Les gommes utilisées dans les compositions selon la présente invention sont choisies de préférence parmi les polyholosides, tels que les carraghénanes ou la gomme de xanthane, et leurs dérivés.

Pour la définition des polymères synthétiques ayant des propriétés gélifiantes, on peut se reporter au document :
- « RESINS, WATER SOLUBLE, Encyclopedia of Chemical Technology, KIRK-OTHMER», vol.20. p.207-230, 3ème édition, 1978. Wiley.

Parmi les polymères synthétiques ayant des propriétés gélifiantes, utilisables selon la présente invention on peut citer en particulier les polymères carboxyvinyliques (carbomer) tels que les produits vendus sous les noms CAR-BOPOL 980, 981, 954, 2984 et 5984 par la société GOODRICH ou les produits vendus sous les noms SYNTHALEN M et SYNTHALEN K par la société 3 VSA.

Les émulsions objet de l'invention comprennent de 0,1 à 2% d'un système gélifiant (B), celul-ci comprenant de préférence au moins 0,05 % par rapport à la composition finale, d'au moins un polymère émulsionnant.

Les compositions selon l'invention peuvent en outre comprendre un ou plusieurs tensioactifs, en une quantité inférieure à 2 % en poids, de préférence en quantité inférieure à 1,5 % en poids. Dans ce cas le tensioactif peut être choisi parmi toutes les classes de tensioactifs connues. On peut se reporter au document « Encyclopedia of Chemical Technology, KIRK-OTHMER», volume 22, p.333-432, 3ème édition, 1979, WILEY, où sont citées les principales classes de tensioactifs connues de l'homme du métier ainsi que leurs fonctions (en particulier le fait d'être moussant).

Avantageusement, lorsque l'on introduit un tensioactif dans les compositions selon l'invention, on choisit un tensioactif moussant. Bien que ron obtienne une mousse très satisfaisante en l'absence de tensioactif, la présence d'un tensioactif moussant peut favoriser la formation d'une mousse plus aérée et volumineuse.

Les tensioactifs moussants utilisés selon la présente invention sont choisis parmi les tensioactifs possédant un pouvoir moussant caractérisé par une hauteur de mousse supérieure à 100 mm ; de préférence, supérieure à 120 mm mesurée selon la méthode ROSS-MILES pour une solution à 0,1 % en poids de tensioactif dans de l'eau distillée à 25° C. Ils peuvent être présents à raison de 0-2 % en poids, de préférence 0.5-1,5 % en poids par rapport au poids total de la composition.

La nature de la phase grasse rentrant dans la composition des émulsions selon l'invention n'est pas critique et elle peut ainsi être constituée par tous les composés qui sont déjà connus de façon générale comme convenant pour la fabrication d'émulsions de type huile-dans-eau. En particulier, ces composés peuvent être choisis, seuls ou en mélanges, parmi les différents corps gras, les huiles d'origine végétale, animale ou minérale, les cires naturelles ou synthétiques, et analogues.

On peut citer parmi les huiles utilisables dans la présente invention les huiles d'origine végétale ou animale, comme par exemple le perhydrosqualène, le squalane, l'huile de coprah, l'huile de macadamia, l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide : des huiles d'hydrocarbures telles que des huiles de paraffine, la vaseline ; des huiles de silicone comme les polyméthylsiloxanes, les polyméthylphénylsiloxanes, des polysiloxanes modifiés par des acides gras, des polysiloxanes modifiés par des alcools gras, des polysiloxanes modifiés par des polyoxyalkylènes, des silicones fluorées : des huiles perfluorées et/ou organofluorées; des acides gras supérieurs tels que l'acide myristique, l'acide palmitique, l'acide stéarique, l'acide béhénique, l'acide oléique, l'acide linoléique, l'acide linolénique, l'acide isostéarique, des alcools gras supérieurs tels que le cétanol, l'alcool stéarylique, l'alcool oléique ; des mono et des di-esters parmi lesquels on peut citer en particulier le myristate d'isopropyle, le palmitate d'isopropyle, le stéarate de butyle, le laurate d'hexyle, l'isononanoate d'isononyle, le palmitate de 2-éthyl hexyle, le laurate de 2-hexyl décyle, le palmitate de 2-octyl décyle, le myristate de 2-octyl dodécyle, le succinate de 2-diethylhexyle, le malate de diisostéaryle, le lactate de 2-octyl dodécyle, le triisostéarate de glycérine, le triisostéarate de glycérine, l'adipate de di-n-butyle, l'adipate de di-(2-éthyl hexyle), le dioléate d'éthylène glycol, le di-isotridécanoate d'éthylène glycol, le di-isostéarate d'éthylène glycol, le di-caprylate de néopentylglycol.

Bien entendu, la phase grasse peut également contenir un ou plusieurs adjuvants cosmétiques lipophiles classiques.

La phase grasse représente 10 à 30 % du poids total de la composition.

La phase grasse peut être constituée uniquement d'huile cosmétique ; elle peut également comprendre des cires animales végétales ou synthétiques, de préférence en quantités inférieures à 1 %.
De préférence, les compositions selon l'invention comprennent de 10 à 30% en poids par rapport au poids total de la composition, d'au moins une huile cosmétique.

Les émulsions selon l'invention comprennent de l'eau. Habituellement on entend par eau de l'eau déminéralisée. Toutefois, une partie de l'eau utilisée dans les émulsions selon l'invention peut éventuellement être choisie parmi les eaux minérales ou thermales. En général, une eau minérale est propre à la consommation, ce qui n'est pas toujours le cas d'une eau thermale. Chacune de ces eaux contient, entre autre, des minéraux solubilisés et des oligo-éléments. Ces eaux sont connues pour être employées à des fins de traitement spécifique selon les oligo-éléments et les minéraux particuliers qu'eues contiennent, tels que l'hydratation et la désensibilisation de la peau ou le traitement de certaines dermatoses. Par eaux minérales ou thermales, on désignera non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou oLigo-élémentaires préparées à partir d'eau purifiée (déminéralisée ou distillée).

Une eau thermale ou minérale naturelle utilisée selon l'invention peut, par exemple être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains.

De manière classique, la phase aqueuse dispersante peut être constituée par de l'eau, ou un mélange d'eau et d'alcool(s) polyhydrique(s) comme par exemple glycérol, propylène glycol et sorbitol, ou bien encore un mélange d'eau et d'alcool(s) inférieur(s) hydrosoluble(s) tels que éthanol, isopropanol ou butanol (solution hydroalcoolique), et elle peut bien entendu en outre contenir des adjuvants cosmétiques classiques hydrosolubles.

Les émulsions cosmétiques ou dermatologiques de l'invention peuvent, en outre, contenir des adjuvants hydrosolubles ou liposolubles habituels dans le domaine cosmétique tels que les conservateurs, les antioxydants, les parfums, les filtres, les matières colorantes, les actifs hydrophiles ou lipophiles.

Les actifs pour la peau peuvent être des actifs anti-àge, des actifs antirides, des hydratants ou des humectants, des actifs amincissants, des actifs dépigmentants, des actifs arti-radicaux libres (espèces radicalaires de l'oxygène), des actifs nutritifs, des actifs protecteurs, des actifs restructurant, des actifs raffermissants, des actifs antiacnéiques, des actifs exfoliants, des actifs émollients ou encore des actifs traitants des maladies de peau comme les mycoses, les dermites, le psoriasis, etc. Ces actifs sont utilisés, selon leur nature, dans les proportions habituelles des émulsions, et par exemple de 0,01 à 10 % en poids par rapport au poids total de la composition.

Le moyen de propulsion est, par exemple, un gaz propulseur habituellement un dispositif pressurisé selon l'invention contient 0,5 à 20 % de gaz propulseur et 80 à 99,5 % d'émulsion. On peut utiliser dans les dispositifs selon l'invention tous les gaz propulseurs connus pour de telles applications. On peut citer en particulier les gaz hydrocarbonés comme par exemple le propane, l'isopropane le n-butane, l'isobutane, l'isopentane et leurs mélanges ; les gaz fluorés comme par exemple le chlorodifluorométhane, le dichlororodifluorométhane, le difluoroéthane, le chlorodifluoroéthane, le dichlorotétrafluoroéthane, etc. et leurs mélanges ; l'azote et le dioxyde de carbone et leurs mélanges peuvent également être utilisés comme gaz propulseurs dans la présente invention.

De préférence on choisit un gaz ayant un point d'ébullition supérieur ou égal à 30°C, de tels gaz favorisant le phénomène de post-moussage. Préférentiellement de tels gaz sont choisis parmi les hydrocarbures, en particulier l'isopentane, l'isobutane et leurs mélanges.

La composition comprise dans le dispositif de l'invention est utilisée comme composition cosmétique pour application sur la peau et/ou les cheveux, en particulier pour le traitement et/ou le nettoyage et/ou le soin de la peau.

La composition comprise dans le dispositif de l'invention est utilisée pour la fabrication d'une composition dermatologique destinée au traitement et/ou au soin et/ou au nettoyage de la peau et/ou des cheveux.

Les compositions comprises dans les dispositifs de l'invention comprenant peu ou pas du tout de tensioactif sont particulièrement adaptées pour le soin et/ou le traitement et/ou le nettoyage des peaux sensibles.

Le procédé de traitement cosmétique de la peau ou des cheveux consiste à appliquer sur ceux-ci une quantité efficace d'une composition cosmétique comprise dans le dispositif de l'invention.

### EXEMPLES

Les exemples ci-après de dispositifs selon l'invention, sont donnés à titre d'illustration et sans caractère limitatif. Les quantités y sont données en % en poids de matière active par rapport au poids total de la composition, sauf mention contraire.

Les noms de matière première sont les noms CTFA.

### Exemple 1 : Dispositif comprenant une crème démaquillante auto-moussante

| | |
|---|---|
| Huile minérale | 15% |
| C₁₃-C₁₄ isoparaffine | 0,6 % |
| Glycérine | 6 % |
| Sodium laureth sulfate | 1 % |
| Laureth-7 | 0,2 % |
| Polyacrylamide (*) | 1,2% |
| Carbomer | 0,1 % |
| Sodium hydroxyde | 0,04 % |
| Parfum | qs |
| Conservateurs | qs |
| Eau | qsp 100 % |

| | |
|---|---|
| (*) commercialisé sous le nom de marque SEPIGEL par la société SEP-PIC | |

Mode opératoire : On fait gonfler les polymères (Carbomer, polymère acrylique et gommes) dans de l'eau chaude (60°C) sous agitation. On introduit le propylène glycol puis la soude. Après neutralisation par la soude, on introduit les conservateurs. Sous agitation (turbine), l'huile et le parfum sont dispersés pour obtenir une émulsion fisse et brillante.
L'émulsion est introduite dans un récipient aérosol et pressurisée avec 3,5 % d'isopentane.
Par actionnement de la valve du dispositif, celui-ci restitue son contenu sous la forme d'une crème qui se transforme en mousse au contact de la peau.

### Exempte 2 : Dispositif comprenant une crème de toilette auto-moussante pour peaux sensibles

| | |
|---|---|
| Octyl palmitate | 10 % |
| Propylène glycol | 5 % |
| Carbomer | 0,3 % |
| Acrylate/C₁₀-C₃₀ alkylacrylate crosspolymer (*) | 0,2 % |
| Gomme de xanthane | 0,3 % |
| Sodium Hydroxide | 0,18 % |
| Parfum | qs |
| Conservateurs | qs |
| Eau | qsp 100 % |

| | |
|---|---|
| (*) commercialisé sous le nom de marque PEMULEN par la société GOODRICH | |

On prépare une émulsion conformément à l'exemple ci-dessus, puis cette émulsion est introduite dans un récipient aérosol et pressurisée avec 3,5 % disopentane. Par actionnement de la valve du dispositif, celui-ci restitue son contenu sous la forme d'une crème qui se transforme
en mousse fine dense et onctueuse par l'effet du massage sur la peau.

### Exemple 3 : dispositif comprenant une crème de toilette auto-moussante

| | |
|---|---|
| Octyl palmitate | 10 % |
| Huile minérale | 10 % |
| Sodium Laureth Sulfate | 1,5 % |
| Propylène glycol | 3 % |
| Carbomer | 0,6 % |
| Acrytate/C₁₀-C₃₀ alkylacrylate crosspolymer (*) | 0,2 % |
| Sodium Hydroxide | 0,35 % |
| Parfum | qs |
| Conservateurs | qs |
| Eau | qsp 100 % |

| | |
|---|---|
| (*) commercialisé sous le nom de marque PEMULEN par la société GOODRICH | |

On prépare une émulsion conformément à l'exemple 1, puis cette émulsion est introduite dans un récipient aérosol et pressurisée avec 3,5 % d'isopentane. Par actionnement de la valve du dispositif, celui-ci restitue son contenu sous la forme d'une crème qui se transforme en mousse fine dense et onctueuse par l'effet du massage sur la peau.

## Revendications

1. Dispositif aérosol constitué d'un récipient pressurisable muni d'un moyen de diffusion comprenant une valve, ledit récipient comprenant un moyen de propulsion et une composition auto-moussante, sous la forme d'une émulsion huile-dans-eau, pressurisée et conditionnée dans le récipient aérosol, à post-moussage, comprenant :
(A) 10 à 30% en poids par rapport au poids total de la composition, d'une phase grasse comprenant au moins une huile cosmétique ;
(B) de 0,1 à 2% d'un système gélifiant comprenant :
- (B1) au moins un polymère émulsionnant choisi parmi:
(1) les homopolymères réticulés, formés à partir d'au moins un monomère, cationique ou anionique, à insaturation éthylènique choisi parmi le (meth)acrylate d'ammonium; l'acide 2-acrylamido-2-méthylpropane sulfonique ainsi que ses sels; les dialkylaminoalkyl-(meth)acrylates, de préférence le diméthylamino-éthylmethacrylate ainsi que leurs sels quaternaires ou acides ; les dialkylaminoalkyl-(meth)acrylamides ainsi que leurs sels quaternaires ou acides ; les radicaux alkyle étant, de préférence, en C₁-C₄, et d'un agent de réticulation a polyinsaturation éthylénique,
(2) les copolymères réticulés formés à partir d'au moins un monomère, cationique ou anionique, à insaturation éthylènique, d'au moins un co-monomère non-ionique et d'un agent de réticulation à polyinsaturation éthylènique, et
- (B2) au moins un gélifiant hydrophile naturel ou synthétique ;
(C) de l'eau.

2. Dispositif selon la revendication précédente, **caractérisé en ce que** la composition comprend 0-1,5% en poids d'au moins un tensioactif.

3. Dispositif selon la revendication 1 ou 2, **caractérisé en ce que** le monomère anionique est choisi parmi l'acide (meth)acrylique, le (meth)acrylate d'amonium et l'acide 2-acrylamido-2-méthylpropane sulfonique ainsi que ses sels.

4. Dispositif selon l'une quelconque des revendications précédentes, **caractérisé en ce que** le monomère cationique est choisi parmi les dialkylaminoakyl-(meth)acrylates, de préférence le diméthylamino-éthylmethacrylate ; les dialkyl-aminoalkyl-(meth)acrylamides ainsi que leurs sels quaternaires ou acides; les radicaux alkyle étant, de préférence, en C₁-C₄.

5. Dispositif selon l'une quelconque des revendications précédentes, **caractérisé en ce que** le co-monomère non-ionique est choisi parmi le méthacrylamide, l'acrylamide, les esters d'acide (meth)acrylique en C₁₀-C₃₀ et les esters de vinyle.

6. Dispositif selon l'une quelconque des revendications précédentes, **caractérisé en ce que** les agents de réticulation à polyinsaturation éthylénique sont choisi parmi le divinylbenzène, le tétraallyloxyéthane ; l'éther diallylique ; les éthers polyallyliques polyglycéryliques ; les ethers allyliques d'alcools de la série du sucre comme l'érythrytol, le pentaérythrytol, l'arabitol, le sorbitol ou le glucose; le méthylène-bis-acrylamide, l'éthylèneglycol di(méthyl)acrylate, le di(meth)acrylamide, le cyano-méthylacrylate, le vinyloxyéthyl-(meth)acrylate ou leurs sels métalliques.

7. Dispositif selon l'une quelconque des revendications précédentes, **caractérisé en ce que** le polymère émulsionnant est choisi parmi :
(a) les copolymères comportant une fraction majoritaire d'acide acrylique et une faible fraction d'esters d'acide (meth)acrylique en C₁₀-C₃₀ ;
(b) les copolymères d'acide 2-acrylamido-2-méthyl-propane sulfonique partiellement ou totalement neutralisé et d'acrylamide ;
(c) les homopolymères de diméthylaminoéthyl-méthacrylate quaternisé par le chlorure de méthyle ou les copolymères de ce même monomère et d'acrylamide ;
(d) les copolymères d'acide(méth)acrylique et d'esters de vinyle.

8. Dispositif selon l'une des revendications précédentes, **caractérisé en ce que** le gélifiant hydrophile est choisi parmi les gommes naturelles ou modifiées et les polymères synthétiques hydrophiles.

9. Dispositif selon la revendication 8, **caractérisé en ce que** le gélifiant hydrophile est choisi parmi la gomme de xanthane, les carraghénanes et leurs dérivés, les polymères carboxyvinyliques.

10. Dispositif selon l'une quelconque des revendications précédentes, **caractérisé en ce que** le système gélifiant (B) comprend au moins 0,05% en poids par rapport à la composition finale, d'au moins un polymère émulsionnant.

11. Dispositif selon l'une quelconque des revendications précédentes, **caractérisé en ce que** la composition comprend au moins un tensioactif moussant.

12. Dispositif selon la revendication 11, **caractérisé en ce que** le tensioactif moussant possède un pouvoir moussant déterminé par une hauteur de mousse supérieure à 100 mm mesurée selon la méthode ROSS-MILES pour une solution à 0,1% en poids de tensioactifs dans de l'eau distillée à 25°C.

13. Dispositif selon l'une quelconque des revendications précédentes, **caractérisée en ce que** la phase grasse est constituée uniquement d'au moins une huile cosmétique.

14. Dispositif selon l'une quelconque des revendications précédentes, **caractérisé en ce que** la composition comprend au moins une eau minérale ou thermale.

15. Dispositif selon la revendication 14, **caractérisé en ce que** l'eau thermale ou minérale est choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-Bains.

16. Dispositif selon l'une quelconque des revendications précédentes, **caractérisé en ce que** la composition comprend de 0,01 à 10% en poids par rapport au poids total de la composition, d'au moins un adjuvant hydrosoluble ou liposoluble cosmétique choisi parmi les conservateurs, les antioxydants, les parfums, les filtres, les matières colorantes, les actifs hydrophiles ou lipophiles.

17. Dispositif selon l'une quelconque des revendications précédentes, **caractérisé en ce qu'**il contient 0,5 à 20% de gaz propulseur comme moyen de propulsion et 80 à 99,5% de composition.

18. Dispositif selon l'une quelconque des revendications précédentes, **caractérisé en ce que** le moyen de propulsion est choisi parmi l'isopentane, l'isobutane et leurs mélanges.

## Claims

1. Aerosol device consisting of a pressurizable container fitted with a diffusion means comprising a valve, the said container comprising a propulsion means and a self-foaming composition in the form of a post-foaming, oil-in-water emulsion, pressurized and packaged in the aerosol container comprising:
(A) 10 to 30% by weight, relative to the total weight of the composition, of a fatty phase comprising at least one cosmetic oil,
(B) from 0.1 to 2% of a gelling system comprising:
- (B1) at least one emulsifying polymer chosen from :
(1) crosslinked homopolymers formed from at least one cationic or anionic monomer containing ethylenic unsaturation selected from the group consisting of ammonium (meth)acrylate; 2-acrylamido-2-methylpropanesulfonic acid and its salts, dialkylaminoalkyl (meth)acrylates, preferably dimethylaminoethyl methacrylate and their quaternary salts or acids; dialkylaminoalkyl (meth)acrylamide and their quaternary salts or acids ; the alkyl radicals preferably being C₁-C₄ and a crosslinking agent containing ethylenic polyunsaturation, and
(2) crosslinked copolymers formed from at least one cationic or anionic monomer containing ethylenic unsaturation, from at least one nonionic comonomer and from a crosslinking agent containing ethylenic polyunsaturation,
- (B2) at least one natural or synthetic hydrophilic gelling agent ; and
(C) water.

2. Device according to the preceding claim, **characterized in that** the composition comprises 0 - 1.5% by weight of at least one surfactant.

3. Device according to the claim 1 or 2, **characterized in that** the anionic monomer is selected from the group consisting of (meth)acrylic acid, ammonium (meth)acrylate and 2-acrylamido-2-methylpropanesulfonic acid, and salts thereof.

4. Device according to any one of the preceding claims, **characterized in that** the cationic monomer is selected from the group consisting of dialkylaminoalkyl (meth)acrylates, preferably dimethylaminoethyl methacrylate; dialkylaminoalkyl (meth)acrylamides and its quaternary salts or acid; the alkyl radicals preferably being C₁-C₄.

5. Device according to any one of the preceding claims, **characterized in that** the nonionic comonomer is selected from the group consisting of methacrylamide, acrylamide, C₁₀-C₃₀ (meth)acrylic acid esters and vinyl esters.

6. Device according to any one of the preceding claims, **characterized in that** the crosslinking agents containing ethylenic polyunsaturation are selected from the group consisting of divinylbenzene, tetraallyloxyethane, diallyl ether, polyallyl polyglyceryl ethers, allylic ethers of alcohols of the sugar series such as erythritol, pentaerythritol, arabitol, sorbitol and glucose; methylenebisacrylamide, ethylene glycol di(methyl)acrylate, di(meth)acrylamide, cyanomethyl acrylate and vinyloxyethyl (meth)acrylate or their metal salts.

7. Device according to any one of the preceding claims, **characterized in that** the emulsifying polymer is selected from the group consisting of:
(a) copolymers containing a major fraction of acrylic acid and a minor fraction of C₁₀-C₃₀ (meth)acrylic acid esters;
(b) copolymers of partially or totally neutralized 2-acrylamido-2-methylpropanesulfonic acid and of acrylamide;
(c) homopolymers of dimethylaminoethyl methacrylate quaternized with methyl chloride or copolymers of this same monomer and of acrylamide; and
(d) copolymers of (meth)acrylic acid and of vinyl esters.

8. Device according to one of the preceding claims, **characterized in that** the hydrophilic gelling agent is selected from the group consisting of natural or modified gums and hydrophilic synthetic polymers.

9. Device according to claim 8, **characterized in that** the hydrophilic gelling agent is selected from the group consisting of xanthan gum, carrageenans and their derivatives, and carboxyvinylpolymers.

10. Device according to any one of the preceding claims, **characterized in that** the gelling system (B) comprises at least 0.05% by weight, relative to the final composition, of at least one emulsifying polymer.

11. Device according to any one of the preceding claims, **characterized in that** the composition comprises at least a foaming surfactant.

12. Device according to claim 11, **characterized in that** the foaming surfactant has a foaming power determined by a foam height of more than 100 mm, measured according to the Ross-Miles method for a 0.1% by weight solution of surfactant in distilled water at 25°C.

13. Device according to any one of the preceding claims, **characterized in that** the fatty phase consist solely of cosmetic oil fatty.

14. Device according to any one of the preceding claims, **characterized in that** the composition comprises at least one mineral water or spring water.

15. Device according to claim 14, **characterized in that** the spring or mineral water is selected from the group consisting of Eau de Vittel, the waters from the Vichy basin, Eau d'Uriage, Eau de la Roche Posay, Eau de la Bourboule, Eau d'Enghien-les-Bains, Eau de Saint Gervais-les-Bains, Eau de Neris-les-Bains, Eau d'Allevar-les-Bains, Eau de Digne, Eau de Maizieres, Eau de Neyrac-les-Bains, Eau de Lons-le-Saunier, les Eaux Bonnes, Eau de Rochefort, Eau de Saint Christau, Eau des Fumades and Eau de Tercis-les-bains.

16. Device according to any one of the preceding claims, **characterized in that** the composition comprises from 0.01 to 10% by weight, relative to the total weight of the composition, of at least one cosmetic water-soluble or liposoluble adjuvant selected from the group consisting of preserving agents, antioxidants, fragrances, screening agents, dyestuffs, and hydrophilic or lipophilic active agents.

17. Device according to any one of the preceding claims, **characterized in that** it contains 0.5 to 20% of propellant gas as propulsion means and 80 to 99.5% of composition.

18. Device according to any one of the preceding claims, **characterized in that** the propulsion means is isopentane, isobutane or mixtures thereof.

## Patentansprüche

1. Aerosolvorrichtung, die aus einem Druckbehälter besteht, der mit einer Verteilervorrichtung ausgestattet ist, die ein Ventil aufweist, wobei der Behälter ein Treibmittel und eine selbstschäumende Zusammensetzung in Form einer Öl-in-Wasser-Emulsion enthält, die in dem Aerosolbehälter unter Druck gesetzt und konditioniert ist und nachschäumend ist, enthaltend:
(A) 10 bis 30 Gew.-% einer Fettphase, die mindestens ein kosmetisches Ö1 enthält, bezogen auf das Gesamtgewicht der Zusammensetzung;
(B) 0,1 bis 2% eines gelbildenden Systems, enthaltend:
- (B1) mindestens ein emulgierendes Polymer, das ausgewählt ist unter:
(1) vernetzten Homopolymeren, die aus mindestens einem kationischen oder anionischen, ethylenisch ungesättigten Monomer, das unter Ammonium(meth)acrylat; 2-Acrylamido-2-methylpropansulfonsäure sowie den Salzen dieser Säure; Dialkylaminoalkyl(meth)acrylaten, vorzugsweise Dimethylaminoethylmethacrylat, sowie den quartären Salzen oder Säureadditionssalzen dieser Verbindungen; Dialkylaminoalkyl(meth)acrylamiden sowie den quartären Salzen oder Säureadditionssalzen dieser Verbindungen ausgewählt ist, wobei die Alkylgruppen vorzugsweise ein bis vier Kohlenstoffatome aufweisen, und einem mehrfach ethylenisch ungesättigten Vernetzungsmittel gebildet sind, und
(2) vernetzten Copolymeren, die aus mindestens einem kationischen oder anionischen, ethylenisch ungesättigten Monomer, mindestens einem nichtionischen Comonomer und einem mehrfach ethylenisch ungesättigten Vernetzungsmittel gebildet sind, und
- (B2) mindestens einen natürlichen oder synthetischen hydrophilen Gelbildner;
(C) Wasser.

2. Vorrichtung nach dem vorhergehenden Anspruch, **dadurch gekennzeichnet, dass** die Zusammensetzung 0-1,5 Gew.-% mindestens eines grenzflächenaktiven Stoffs enthält.

3. Vorrichtung nach Anspruch 1 oder 2, **dadurch gekennzeichnet, dass** das anionische Monomer ausgewählt ist unter (Meth)acrylsäure, Ammonium(meth)acrylat und 2-Acrylamido-2-methylpropansulfonsäure sowie den Salzen dieser Säure.

4. Vorrichtung nach einem der vorhergehenden Ansprüche, **dadurch gekennzeichnet, dass** das kationische Monomer ausgewählt ist unter Dialkylaminoalkyl(meth)acrylaten, vorzugsweise Dimethylaminoethylmethacrylat, und Dialkylaminoalkyl(meth)acrylamiden sowie den quartären Salzen oder Säureadditionssalzen dieser Verbindungen, wobei die Alkylgruppen vorzugsweise ein bis vier Kohlenstoffatome aufweisen.

5. Vorrichtung nach einem der vorhergehenden Ansprüche, **dadurch gekennzeichnet, dass** das nicht-ionische Comonomer ausgewählt ist unter Methacrylamid, Acrylamid, C₁₀₋₃₀-(Meth)acrylsäureestern und Vinylestern.

6. Vorrichtung nach einem der vorhergehenden Ansprüche, **dadurch gekennzeichnet, dass** die mehrfach ethylenisch ungesättigten Vernetzungsmittel ausgewählt sind unter Divinylbenzol, Tetraallyloxyethan, Diallylether, Polyallylpolyglycerylethern, Allylethern von Alkoholen aus der Zuckerreihe, wie Erythrit, Pentaerythrit, Arabit, Sorbit oder Glucose, Methylen-bisacrylamid, Ethylenglykol-di(methyl)acrylat, Di(meth)acrylamid, Cyanomethylacrylat, Vinyloxyethyl(meth)acrylat oder den Metallsalzen dieser Verbindungen.

7. Vorrichtung nach einem der vorhergehenden Ansprüche, **dadurch gekennzeichnet, dass** das emulgierende Polymer ausgewählt ist unter:
(a) den Copolymeren, die einen hohen Anteil an Acrylsäure und einen geringeren Anteil an C₁₀₋₃₀-(Meth)acrylsäureestern enthalten;
(b) den Copolymeren von zum Teil oder vollständig neutralisierter 2-Acrylamido-2-methylpropansulfonsäure und Acrylamid;
(c) den Homopolymeren von mit Methylchlorid quarternisiertem Dimethylaminoethylmethacrylat oder den Copolymeren dieses Monomers und Acrylamid und
(d) den Copolymeren von (Meth)acrylsäure und Vinylestern.

8. Vorrichtung nach einem der vorhergehenden Ansprüche, **dadurch gekennzeichnet, dass** der hydrophile Gelbildner unter den natürlichen oder modifizierten Gummen und hydrophilen synthetischen Polymeren ausgewählt ist.

9. Vorrichtung nach Anspruch 8, **dadurch gekennzeichnet, dass** der hydrophile Gelbildner unter Xanthangummi, Carrageenanen und ihren Derivaten und Carboxyvinylpolymeren ausgewählt ist.

10. Vorrichtung nach einem der vorhergehenden Ansprüche, **dadurch gekennzeichnet, dass** das gelbildende System (B) bezogen auf die Endzusammensetzung mindestens 0,05 Gew.-% mindestens eines emulgierenden Polymers enthält.

11. Vorrichtung nach einem der vorhergehenden Ansprüche, **dadurch gekennzeichnet, dass** die Zusammensetzung mindestens einen schäumenden grenzflächenaktiven Stoff enthält.

12. Vorrichtung nach Anspruch 11, **dadurch gekennzeichnet, dass** der schäumende grenzflächenaktive Stoff ein Schäumungsvermögen aufweist, das nach dem ROSS-MILES-Verfahren bei 25°C an einer Lösung mit 0,1 Gew.-% grenzflächenaktiven Stoffen in destilliertem Wasser mit einer Schaumhöhe von über 100 mm bestimmt wurde.

13. Vorrichtung nach einem der vorhergehenden Ansprüche, **dadurch gekennzeichnet, dass** die Fettphase nur aus mindestens einem kosmetischen Öl besteht.

14. Vorrichtung nach einem der vorhergehenden Ansprüche, **dadurch gekennzeichnet, dass** die Zusammensetzung mindestens ein Mineralwasser oder Thermalwasser enthält.

15. Vorrichtung nach Anspruch 14, **dadurch gekennzeichnet, dass** das Thermalwasser oder Mineralwasser ausgewählt ist unter Wasser aus Vittel, Wässern aus der Vichy-Ebene, Wasser aus Uriage, Wasser aus Roche Posay, Wasser aus Bourboule, Wasser aus Enghien-les-Bains, Wasser aus Saint Gervais-les-Bains, Wasser aus Neris-les-Bains, Wasser aus Allevar-les-Bains, Wasser aus Digne, Wasser aus Maizières, Wasser aus Neyrac-les-Bains, Wasser aus Lons-le-Saunier, Bonnes-Wässern, Wasser aus Rochefort, Wasser aus Saint Christau, Wasser aus Fumades und Wasser aus Tercis-les-Bains.

16. Vorrichtung nach einem der vorhergehenden Ansprüche, **dadurch gekennzeichnet, dass** die Zusammensetzung, bezogen auf das Gesamtgewicht der Zusammensetzung, 0,01 bis 10 Gew.-% mindestens eines wasserlöslichen oder fettlöslichen kosmetischen Zusatzstoffs enthält, der unter Konservierungsmitteln, Antioxidantien, Parfums, Filtern, Färbemitteln und hydrophilen oder lipophilen Wirkstoffen ausgewählt ist.

17. Vorrichtung nach einem der vorhergehenden Ansprüche, **dadurch gekennzeichnet, dass** sie 0,5 bis 20% Treibgas als Treibmittel und 80 bis 99,5% Zusammensetzung enthält.

18. Vorrichtung nach einem der vorhergehenden Ansprüche, **dadurch gekennzeichnet, dass** das Treibmittel unter Isopentan, Isobutan und den Gemischen dieser Verbindungen ausgewählt ist.
